# EUROPÄISCHE PATENTANMELDUNG

(11) **EP 0 759 307 A2**
(43) Veröffentlichungstag der Anmeldung: **26.02.1997**
(21) Anmeldenummer: 96113019.2
(22) Anmeldetag: 13.08.1996
(51) Int. Cl.: A61N 1/34, A61N 1/05

(54) **Punktier- und/oder Katheterisiervorrichtung zum Herantasten an Nerven**

(30) Priorität: 22.08.1995 DE 19530869
(71) Anmelder: STERIMED Medizinprodukte GmbH, D-66346 Püttlingen (DE)
(72) Erfinder: März, Peter, Dr., 82377 Penzberg (DE)
(74) Vertreter: Alber, Norbert

(57) **Zusammenfassung**

Die Anmeldung beschreibt eine Punktier- und/oder Katheterisiervorrichtung mit einer zum Herantasten an den Nerv zwischen Gefäßnervenscheide und Nerv einzustechenden Punktiernadelelektrode, die Bestandteil eines Stimulatorstromkreises ist, dessen nacheinander abgegebene Stimulator-Impulse im Laufe der Impulsfolge in ihrer Stärke in Richtung auf kleinere Werte veränderbar sind und für den Nervensuchvorgang vom Behandlungspersonal hinsichtlich der von ihnen hervorgerufenen Muskelkontraktionen zu beobachten sind. Dabei ist eine Diagnostiziervorrichtung vorgesehen, die nicht nur die Tatsache einer Gefäßdurchblutung überhaupt anzeigt, sondern das von ihr ausgesendete Signal entsprechend der Lage ihrer Sonde gegenüber dem festgestellten Durchblutungsstrom verändert. Dabei ist das Gerät mit einer Ultraschallsonde, insbesondere Doppler-Sonde als Diagnostiziervorrichtung ausgestattet und die Punktiernadelelektrode hat eine stumpfe Kanüle.

## Beschreibung

Die Erfindung betrifft eine Punktier- und/oder Katheterisiervorrichtung mit einer zum Herantasten an den Nerv zwischen Gefäßnervenscheide und Nerv einzustechenden Punktiernadelelektrode, die Bestandteil eines Stimulatorstromkreises ist, dessen nacheinander abgegebene Stimulatorimpulse im Laufe der Impulsfolge in ihrer Stärke in Richtung auf kleinere Werte veränderbar sind und für den Nervensuchvorgang vom Behandlungspersonal hinsichtlich der von ihnen hervorgerufenen Muskelkontraktionen zu beobachten sind.

Punktier- und/oder Katheterisiervorrichtungen der vorgenannten Art sind seit mehr als 80 Jahren bekannt (Münchener Medizinische Wochenschrift, Jahrgang 1912, Nr. 47, Seite 2545).

Diese Punktier- und/oder Katheterisiervorrichtungen, wie sie in modernerer Version auch aus der EP 298 268 B1 bekannt sind, sollen es dem behandelnden Arzt ermöglichen, die Punktiernadel möglichst schonend und schnell an den Nerv heranzuführen, um dann eine gewünschte Behandlung wie Einführung von Medikamenten, Katheterisierung o.dgl. durchzuführen. Die bekannten Geräte basieren auf dem Prinzip, daß die Stärke der aufeinander folgenden Stimulationsimpulse fortschreitend zurückgenommen und dabei die an dem Patienten auftretenden Muskelkontraktionen beobachtet werden. Wenn selbst bei nur noch geringer Stärke der Stimulationsimpulse Muskelkontraktionen (also beispielsweise ein Zucken der Finger bei im Achselbereich des Patienten eingestochener Punktiernadelektrode) festgestellt werden, ist das bei richtiger Arbeitsweise des Gerätes ein Anhaltspunkt für den behandelnden Arzt, daß er mit der Spitze der Nadel zwischen Gefäßnervenscheide und Nerv dicht am Nerv ist.

Während der vorerwähnte Nervensuchvorgang nach erfolgtem Einführen der Punktiernadelelektrode in die Gefäßnervenscheide mit modernen Geräten bereits sehr schonend und zügig ausgeführt werden kann, besteht nach wie vor eine Problematik darin, beim ersten Einstechen der Punktiernadelelektrode richtig in die das betreffende Nervenbündel als Hülle umgebende Gefäßnervenscheide hineinzutreffen.

Zum Zwecke der Punktion von Gefäßen in Form von Arterien oder Venen ist die sogenannte Sonographie, die sich den Doppler-Effekt zum Orten eines Gefäßes zu Nutze macht, seit mehreren Jahrzehnten bekannt. Die sonographische Gefäßortung erfolgt mit dem Ziel, zielgenau mit einer Punktionsnadel in das geortete Gefäß einzudringen, um z. B. Blut zu entnehmen. Dabei wird das betroffene Gefäß natur- und bestimmungsgemäß verletzt.

Da das Verletzen eines in der Gefäßnervenscheide verlaufenden Gefäßes, etwa einer Arterie, zu einer absolut unerwünschten Blutung innerhalb der mechanisch relativ festen Gefäßnervenscheide führen würde und dadurch die in ihr verlaufenden Nerven mit einem relativ hohen Druck beaufschlagt werden würden, was zum Absterben des Nervs führen kann, wurde bislang von der Anwendung der Sonographie zum Zwecke der Ortung der Gefäßnervenscheide abgesehen. Die herrschende Lehrmeinung vertritt sogar die Auffassung, bei der Nervenstimulation nicht auf eine Arterie zu zielen, sondern neben ihr einzustechen. Darüber hinaus kann mit Hilfe der Sonographie der Nerv selbst nicht erkannt werden und die Punktiernadel könnte beim Vorschieben in Richtung Arterie den Nerv, der in unmittelbarer Nähe der Arterie liegt, verletzen.

Es ist daher die Aufgabe der vorliegenden Erfindung, eine Punktier- und/oder Katheterisiervorrichtung zum Herantasten an Nerven zu schaffen, mit deren Hilfe das erste Einstechen einer Punktiernadelelektrode in die das betreffende Nervenbündel führende Gefäßnervenscheide zielgenau ermöglicht werden soll. Die Lösung der Aufgabe liegt darin, daß die Vorrichtung zusätzlich mit einer auf Gefäßdurchblutung ansprechenden, verletzungsfreien Diagnostiziervorrichtung ausgerüstet ist, die im Bereich der Nerven äußerlich ansetzbar ist.

Die Erfindung nutzt die Tatsache, daß Nervenbündel innerhalb einer gemeinsamen Fascienhülle von durchbluteten Arterien begleitet sind und daß die Feststellung des Schwerpunktes des Durchblutungsstromes dem behandelnden Arzt Anzeichen dafür sein kann, in welcher Richtung sich der von ihm gesuchte Nerv im Körperinneren befindet. Er kann dann seine Punktiernadelelektrode unmittelbar in dieser Richtung einstechen, um dann nach Eindringen in die Gefäßnervenscheide den weiteren Nervensuchvorgang in bekannter Weise mit stufenweiser Herabsetzung der Impulsstärke des Stimulatorstromkreises auszuführen.

Diagnostiziervorrichtungen für ausreichende Gefäßdurchblutung sind an sich bekannt, insbesondere in Form der auch seit vielen Jahren bekannten Doppler-Sonden. Beispiele sind die unter den Markennamen "Sonodop" und "handydop" vertrieben und in Prospekten beschriebenen Geräte. Solche Doppler-Sonden werden im Bereich eines zu untersuchenden Gefäßes auf die Haut aufgesetzt und senden ein Ultraschallsignal mit einer Frequenz im Bereich von beispielsweise 2 - 8 MegaHertz aus. Bei Durchblutung des Gefäßes bewirkt der Durchblutungsstrom eine Modulation des ausgesendeten Ultraschallsignales, und die so bewirkte Modulation wird dann an dem Gerät durch ein optisches Wellesignal oder Schallsignal o.dgl. angezeigt. Dieses diente dem behandelnden Arzt bisher als Hinweis dafür, ob das betreffende Gefäß ausreichend durchblutet ist. Solche Geräte waren auch bereits richtungssensitiv ausgestaltet, um feststellen zu können, in welcher Richtung denn ein geortetes Gefäß von Blut durchströmt ist, und je nach Nähe zum Gefäß konnte auch das erzeugte Signal mehr oder minder stark auftreten.

Das Gerät gemäß der vorliegenden Erfindung benutzt nun eine Diagnostiziervorrichtung, die nicht nur die Tatsache einer Gefäßdurchblutung überhaupt anzeigt, sondern das von ihr ausgesendete Signal auch entsprechend der Lage ihrer Sonde gegenüber dem festgestellten Durchblutungsstrom verändert.

Auf diese Weise kann der behandelnde Arzt bei angesetzter Sonde den Mittelpunkt der lokalisierten Arterie feststellen und damit die richtige Einstechrichtung seiner Punktiernadelelektrode exakt bestimmen.

Gemäß bevorzugter Ausführungsform wird das Gerät mit einer Ultraschallsonde, insbesondere Doppler-Sonde als Diagnostiziervorrichtung ausgestattet.

Gemäß einem weiteren Erfindungsmerkmal (Kennzeichen des Anspruchs 4) hat die Punktiernadelelektrode eine stumpfe Kanüle.

Da die vorliegende Erfindung es gestattet, die Kanüle der Punktiernadelelektrode zentral an der Arterie und Nerven enthaltenden Gefäßnervenscheide angreifen zu lassen, ist sichergestellt, daß auch eine stumpfe Kanüle durch radiales Einbeulen der Gefäßnervenscheide in der gewünschten Weise auf den Mittelpunkt des Gefäßes vordringt. Bisher bestand bei Verwendung stumpfer Kanülen die Gefahr eines nicht exakt radialen Ansatzes, sondern mehr tangentialen Ansatzes, und das wiederum führte zu der Gefahr eines Abrutschens der Kanüle von der Gefäßnervenscheide.

Die erwähnte Einbeulung der Gefäßscheide führt auch zu einer gewissen Selbstzentrierung der stumpfen Kanüle, wie sie mit einer scharfen Kanüle nicht erreichbar ist.

Gemäß einer bevorzugten Ausführungsform der Erfindung (Anspruch 5) sind Stimulatorstromkreis und Durchblutungs-Diagnostiziervorrichtung baueinheitlich miteinander vereinigt.

Gemäß vorteilhafter Ausgestaltung haben Bedienungselemente und Signalvorrichtung in der Weise Mehrfachnutzen, daß eine Doppelbetätigung von Bedienungselementen (Ein-/Ausschalter, Intensitätsschalter) dem Ein- und Ausschalten der Durchblutungs-Diagnostiziervorrichtung und der Lautstärke von dessen Schallsignal zugeordnet ist, während eine Einfachbedienung der Bedienungselemente dem Stimulatorstromkreis für Ein- und Ausschalten und Verstärkung und Verringerung der Stimulatorimpulse zugeordnet ist oder umgekehrt

Gemäß bevorzugter Ausführungsform ist die Punktier- und/oder Katheterisiervorrichtung in ihrem Stimulatorstromkreis in bekannter Weise für Iststrommessung in der Körpergewebestrecke zwischen Hautklebeelektrode und Punktiernadelelektrode ausgelegt und mit Stromkonstanthalter ausgestattet.

Gemäß vorteilhafter Ausgestaltung sind Diagnostiziervorrichtung insbesondere Ultraschallsonde bzw. Dopplersonde und Punktiernadelelektrode nach Art eines Zielgerätes zusammengebaut.

Es folgt die Beschreibung der Erfindung anhand von Figuren. Es zeigen
- Fig. 1: eine schematische Darstellung einer Punktier- und/oder Katheterisiervorrichtung einschließlich einer mit Ultraschallsonde ausgestatteten Diagnostiziervorrichtung zur Ortung eines Nerven enthaltenden Gefäßes von außen her und mit einem Nerven-Stimulatorteil mit einer Hautklebeeleketrode und einer Punktiernadeleleketrode;
- Fig. 2a: eine schematische Darstellung der Wirkungsweise einer Ultraschallsonde, die im Bereich eines eine Arterie und Nervenbündel enthaltenden Gefäßes auf die Haut eines Patienten aufgesetzt ist.
- Fig. 2b: eine schematische Darstellung einer durch die Haut des Patienten eingestochenen und die Gefäßnervenscheide etwas einbeulenden Punktiernadelelektrode;
- Fig. 2c: eine schematische Darstellung der durch die Gefäßvervenscheide eingestochenen und in Richtung auf das Nervenbündel vorgeschobenen Punktiernadelelektrode;
- Fig. 3: eine schematische Darstellung einer als Zielgerät ausgestalteten, gelochten und teilbaren Ultraschallsonde mit hindurchgeführter Punktiernadelelektrode;
- Fig. 4: in Draufsicht ein separates Zielgerät mit Einführteilen für eine Ultraschallsonde und für eine Punktiernadelelektrode;
- Fig. 5: eine Schnittdarstellung durch das Zielgerät gemäß Fig. 4.

Anhand der Zeichnungen wird eine Punktier- und/oder Katheterisiervorrichtung zum Herantasten an Nerven beschrieben, die mit einer Hautklebeelektrode 1, einer Punktiernadelelektrode 3, einem Nervenstimulatorteil 5 und einer Ultraschallsonde 7 ausgestattet ist. Die Ultraschallsonde 7 hat Anschluß an einen nur schematisch angedeuteten Ultraschall-Geräteteil 9 und dient als Diagnostiziervorrichtung zur Ortung eines Nervenbündel und Arterie führenden Gefäßes unterhalb der Haut eines Patienten.

Der Nervenstimulatorteil 5 und der Ultraschall-Geräteteil 9 sind baueinheitlich in einem Gehäuse 11 untergebracht, an dessen Außenseite sich die Anschlüsse für die Ultraschallsonde 7 sowie die Hautklebelektrode 1 und die Punktiernadelelektrode 3 befinden.

Die Leitungsverbindungen der letztgenannten Bestandteile sind in der bei 13 gezeigten Weise durchbrochen dargestellt und haben eine ausreichende Länge, so daß Ultraschallsonde, Hautklebeelektrode und Punktiernadelelektrode in der geeigneten Weise beispielsweise an Unterarm und Achselbereich des Patienten angelegt werden können.

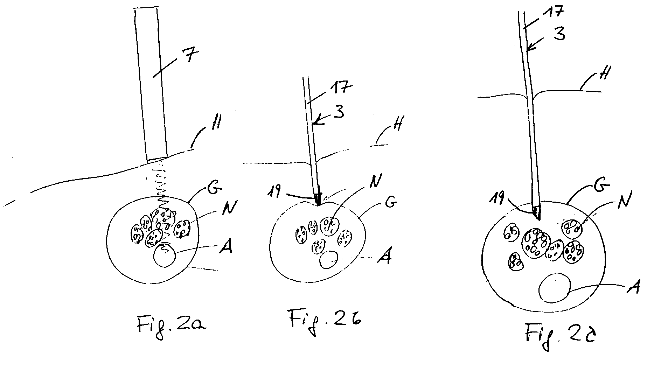

Die Fig. 2a bis 2c veranschaulichen einen Hautbereich H des Patienten mit einem darunter befindlichen Gefäß G, in dem eine Arterie A und Nervenbündel N angedeutet sind.

In der in Fig. 2c angedeuteten Weise soll die Punktiernadelelektrode 3 nach Eindringen in das Gefäß G bis dicht an das Nervenbündel N oder einen Nerven herangeführt werden, um dort beispielsweise für Anästhesiezwecke medizinische Substanzen einzubringen, eine Katheterisierung vorzunehmen, oder dergleichen.

Zu diesem Zweck ist die Punktiernadelelektrode 3 in der üblichen Weise durchgängig und hat ein Anschlußende 15, an welches beispielsweise ein Infusionsbehälter, eine medizinische Spritze o. dgl. anschließbar sind, und es weist die Punktiernadelelektrode 3 über den größten Teil ihrer Länge eine elektrische Isolierung 17 auf, aus der lediglich die abisolierte elektrisch leitende Spitze 19 der Punktiernadelelektrode herausragt. Der elektrisch leitende Teil 19 der Punktiernadelelektrode hat an seinem rückwärtigen Ende Anschluß an die Leitung 21, die zu dem Nervenstimulatorteil 5 der Vorrichtung führt.

Der Nervenstimulatorteil 5 mit der Punktiernadelelektrode 3 und der Hautklebeelektrode 1 sind von bekanntem Aufbau wie in der EP 298 268 B1 beschrieben, und sie gestatten es, sich bei fortschreitend zurückgenommener Stromstärke der Stimulatorimpulse des Stimulatorstromkreises mit der abisolierten Spitze 19 fortschreitend an den Nerven N heranzutasten. In der in der EP 0 298 268 B1 beschriebenen Weise beobachtet dabei der behandelnde Arzt die durch die Stimulatorimpulse hervorgerufenen Muskelkontraktionen und weiß dann, daß er dicht am Nerven N ist, wenn selbst nach Zurücknahme der Stromstärke auf beispielsweise 0,1 Milliamper noch merkliche Muskelkontraktionen zu beobachten sind. Vorzugsweise ist auch im Rahmen der vorliegenden Vorrichtung der Stimulatorstromkreis in der in EP 0 298 268 B1 beschriebenen Weise für Iststrommessung in der Körpergewebestrecke zwischen Hautklebeelektrode 1 und Punktiernadelelektrode 3 ausgestattet.

Um die Ortung des Gefäßes G, bevor die Punktiernadelelektrode 3 durch die Hülle oder Gefäßnervenscheide des Gefäßes eingestochen werden kann, zu erleichtern, ist der Ultraschall-Geräteteil 9 als Diagnostiziervorrichtung für die Lage des Gefäßes vorgesehen. Die Ultraschallsonde 7 ist Bestandteil dieser Diagnostiziervorrichtung und wird in der in Fig. 2a veranschaulichten Weise von dem behandelnden Arzt an der vermuteten Stelle des Gefäßes G auf die Haut des Patienten aufgesetzt. Wenn sich die Ultraschallsonde 7 zentral über dem von der Arterie A durchbluteten Gefäß befindet, ist ein durch den Lautsprecher 21 abgegebenes Schallsignal am stärksten, und der behandelnde Arzt kann dann sicher sein, beim Durchstechen der Haut H mit der Punktiernadelelektrode 3 an dieser Stelle in der in Fig. 2b veranschaulichten Weise zentral auf die die Hülle des Gefäßes G bildende Gefäßnervenscheide zu treffen. Er kann alsdann in der in Fig. 2c gezeigten Weise in die Gefäßnervenscheide 2c einstechen.

Die auf die vorstehende Weise sichergestellte genaue Ortung des Gefäßes G gestattet es auch in der aus medizinischen Gründen erwünschten Weise mit einer stumpfen Punktiernadelelektrode zu arbeiten. Aufgrund der zentralen Ortung des Gefäßes besteht nicht die Gefahr eines nur seitlichen Ansatzes der Punktiernadelelektrode 3 und nachfolgenden Abrutschens. Mit einer stumpfen Punktiernadelelektrode kann der behandelnde Arzt vielmehr gut das in der Literatur bekannte "Klick"-Phänomen mit einem schonenden Eindringen in die Gefäßnervenscheide ausnutzen. Dabei geschieht im übrigen die Ortung des Gefäßes mittels der Ultraschall-Diagnostiziervorrichtung verletzungsfrei, da diese durch die Haut hindurch auf die Gefäßdurchblutung durch die dem Nerven benachbarte Arterie anspricht.

Die technischen Einzelheiten geeigneter Diagnostiziervorrichtungen sind als solche bekannt. Bisher wurden derartige Diagnostiziervorrichtungen zur Ermittlung von Stärke und auch Richtung von Gefäßdurchblutungen eingesetzt, um beispielsweise Durchblutungsstörungen behandeln zu können, Herztöne von Embryonen zu analysieren etc..

Bei dem vorliegenden Ausführungsbeispiel der Erfindung ist als Ultraschallsonde 7 eine Doppler-Sonde vorgesehen, die nach dem in Pschyrembel "Klinisches Wörterbuch", (Verlag De Gruyter, Berlin, New York, 1986) beschriebenen Doppler-Verfahren arbeitet: Ein Kristall sendet kontinuierliche Ultraschallwellen von konstanter Frequenz (Dauerschall) aus. Trifft das Schallwellenbündel auf eine sich bewegende Grenzfläche (vorliegend den Blutstrom innerhalb der den Nerven begleitenden Arterie), so wird ein Teil der Wellen mit geänderter Frequenz (Doppler-Effekt) reflektiert. Die Interferenz der Frequenzen des einfallenden und des reflektierenden Strahles ergibt einen niederfrequenten Ton. Dieser Ton wird durch Verstärkung hörbar gemacht und bei dem vorliegenden Gerät am Lautsprecher 21 ausgesendet.

Die vorliegende Erfindung nutzt die Erscheinung, daß der ausgesendete Ton um so stärker ist, je zentraler sich die Ultraschallsonde 7 über dem Gefäß G befindet, so wie in Fig. 2a angedeutet.

Geeignete bekannte Geräte arbeiten beispielsweise mit einer Ultraschallsonde mit folgender Spezifikation: 8MHz-CW-Sonde, Sendeleistung <50mW/cm²(SATA). Solche geräte sind mittels zweier NiCd-Akkus, 9 V betreibbar. Ein marktgängiges Gerät dieser Art ist unter dem Markennamen "handydop" erhältlich (Kranzbühler, 42665 Solingen). Ein anderes marktgängiges Gerät ist als "MINIDOP 8" von Sonotechnik GmbH, 85570 Markt Schwaben erhältlich.

Die technischen Bestandteile der bekannten Ultraschall-Diagnostiziergeräte lassen sich gemäß der vorliegenden Erfindung für günstige Handhabung in dem Gehäuse 11 baueinheitlich zusammenfassen.

An dem Gehäuse 11 können die Bedienungsteile für den Nervenstimulatorteil 5 und den Ultraschall-Geräteteil 9 getrennt oder zusammengefaßt vorgesehen sein.

Bei dem gezeigten Ausführungsbeispiel sieht man auf der Gerätevorderseite eine Start-/Stoptaste, eine Testtaste sowie eine Plustaste und eine Minustaste für die Milliamper(mA)-Einstellungen des Nervenstimulatorteiles 5. Darüber befindet sich das Display mit Anzeigen 23 für den Strom-Sollwert und 25 für den Strom-Istwert sowie 27 für die Batterieprüfung.

Für den Ultraschall-Geräteteil 9 sind auf der linken Seite des baueinheitlichen Gehäuses 11 eine Start-/Stoptaste für das Ultraschall-Signal und je eine "Laut"-Taste und "Leise"-Taste für das am Lautsprecher 22 erscheinende Modulationssignal vorgesehen.

Statt getrennte Tastengruppen für Nervenstimulatorteil 5 und Ultraschall-Geräteteil 9 vorzusehen, ist es gemäß einer nicht näher gezeigten, abgewandelten Ausführungsform auch möglich, den auf der Gerätevorderseite gezeigten Tasten eine Doppelnutzung zu geben. Dazu kann eine Einfachbetätigung einer bestimmten Taste beispielsweise dem Ultraschall-Geräteteil 9 zugeordnet sein, so daß beispielsweise die Einschaltung des Ultraschall-Geräteteiles mit einer einfachen Betätigung der Start-/Stoptaste auf der Vorderseite des Gerätes erfolgt und dann das Laut-/Leisestellen des Lautsprechers 22 durch einfache Betätigung der Plus-/Minustasten auf der Vorderseite des Gerätes.

Für das Einschalten und die weitere Betätigung des Nervenstimulatorteiles 5 wären dann die auf der Vorderseite des Gerätes gezeigten Tasten jeweils zweifach zu betätigen. Derartige Tastenanordnungen mit Doppelnutzen sind bei elektronischen Geräten im übrigen vielfach bekannt und bedürfen daher im einzelnen keiner Beschreibung.

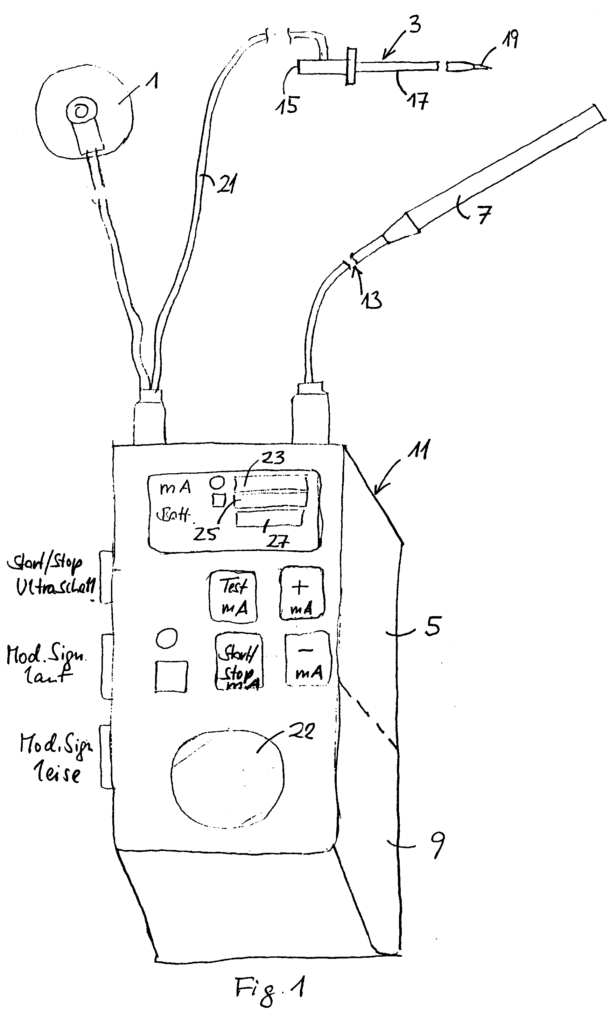

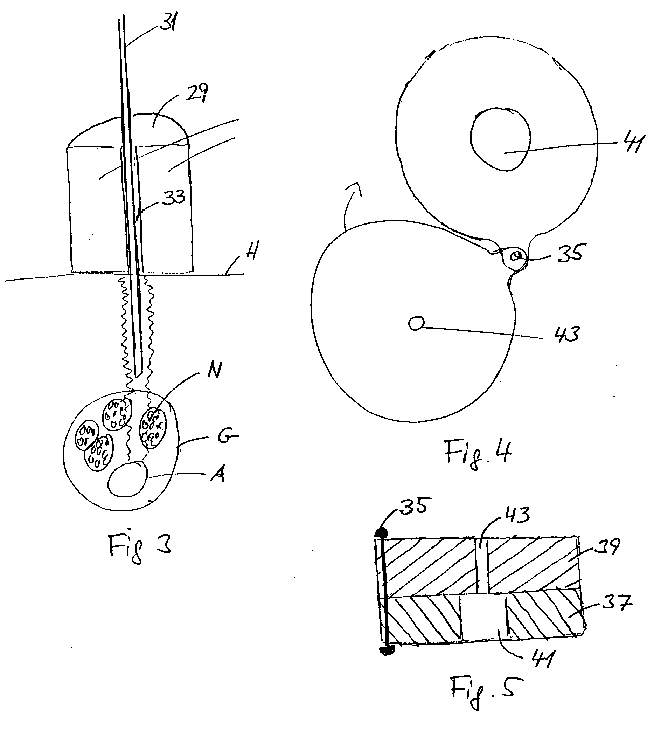

Während in der Fig. 1 eine stabförmige Ultraschallsonde 7 veranschaulicht ist, veranschaulicht die Fig. 3 die eine Hälfte 29 einer teilbaren Ultraschallsonde, die als Zielgerät für eine Punktiernadelelektrode 31 ausgestaltet ist. Die Ultraschallsonde 29 und die Punktiernadelelektrode 31 lassen sich in gleicher Weise wie die Ultraschallsonde 7 und die Punktiernadelelektrode 19 an das in Fig. 1 gezeigte Gerät anschließen, erleichtern dem behandelnden Arzt jedoch die Handhabung weiter: Der behandelnde Arzt setzt zunächst die beiden gleich ausgestalteten Teile 29 der Ultraschallsonde auf die Haut H des Patienten auf, um das Gefäß in der zuvor beschriebenen Weise zu suchen. Dabei dient die Ultraschallsonde mit ihren Teilen 29 als Zielgerät, und die Punktiernadelelektrode 31 befindet sich bereits in dem Kanalteil 33 (aber in noch nicht durch die Haut H eingestochener Lage). Sobald das Gefäß und damit der Nerv mittels der Ultraschallsonde richtig geortet ist, kennt der Arzt die richtige Stichrichtung zum Einstechen und kann dann in der oben erläuterten Weise unter Benutzung des Nervenstimulatorteiles 5 weiter einstechen und sich an den Nerven herantasten.

Die Teilbarkeit der in Fig. 3 schematisch angedeuteten Ultraschallsonde gestattet es dann, die Ultraschallsonde nach Gebrauch vollständig fortzunehmen.

Die Fig. 4 und 5 veranschaulichen eine andere Ausführungsform eines Zielgerätes in Form von zwei mittels eines Gelenkstiftes 35 aneinander angelenkten Scheiben 37 und 39. Die Scheibe 37 hat als Einführteil eine der Stärke beispielsweise der Ultraschallsonde 7 aus Fig. 1 entsprechende Öffnung 41, und die Scheibe 39 eine der Stärke der Punktiernadelelektrode 3 entsprechende Öffnung 43.

Im zusammengeklappten Zustand des Zielgerätes gemäß Fig. 5 sind die beiden Öffnungen 41 und 43 miteinander fluchtend. Für den Ortungsvorgang des Gefäßes wird das Zielgerät gemäß Fig. 4 zunächst auseinandergeklappt und die Ultraschallsonde 7 in die Öffnung 41 eingeführt und auf der Haut des Patienten quer hinund herbewegt, bis die auf dem Gefäß zentrierte Lage erreicht ist. Alsdann wird die Ultraschallsonde 7 herausgenommen, das zielgerät gemäß Fig. 5 zusammengeklappt und es kann alsdann die Punktiernadelelektrode 17 durch die Öffnung 43 an der richtigen Stelle eingeführt und in Richtung auf das Gefäß durch die Haut des Patienten eingestochen werden.

## Patentansprüche

1. Punktier- und/oder Katheterisiervorrichtung mit einer zum Herantasten an den Nerv zwischen Gefäßnervenscheide und Nerv einzustechenden Punktiernadelelektrode, die Bestandteil eines Stimulatorstromkreises ist, dessen nacheinander abgegebene Stimulatorimpulse im Laufe der Impulsfolge in ihrer Stärke in Richtung auf kleinere Werte veränderbar sind und für den Nervensuchvorgang vom Behandlungspersonal hinsichtlich der von ihnen hervorgerufenen Muskelkontraktionen zu beobachten sind,
**dadurch gekennzeichnet, daß**
die Vorrichtung zusätzlich mit einer auf Gefäßdurchblutung ansprechenden, verletzungsfreien Diagnostiziervorrichtung (7) ausgerüstet ist, die im Bereich der Nerven äußerlich ansetzbar ist.

2. Punktier- und/oder Katheterisiervorrichtung nach Anspruch 1,
**gekennzeichnet durch**
eine Diagnostiziervorrichtung ( 7, 9, 21), die nicht nur die Tatsache einer Gefäßdurchblutung überhaupt anzeigt, sondern das von ihr ausgesendete Signal auch entsprechend der Lage ihrer Sonde (7) gegneüber dem festgestellten Durchblutungsstrom veräntert.

3. Punktier- und/oder Katheterisiervorrichtung nach Anspruch 1,
**dadurch gekennzeichnet, daß**
das Gerät mit einer Ultraschallsonde, insbesondere Doppler-Sonde als Diagnostiziervorrichtung ausgestattet ist.

4. Punktier- und/oder Katheterisiervorrichtung nach Anspruch 1,
**dadurch gekennzeichnet, daß**
die Punktiernadelelektrode eine stumpfe Kanüle (19) hat.

5. Punktier- und/oder Katherisiervorrichtung nach einem der voranstehenden Ansprüche,
**dadurch gekennzeichnet, daß**
Stimulatorstromkreis (5) und Durchblutungs-Diagnostiziervorrichtung (9) baueinheitlich miteinander vereint sind.

6. Punktier- und/oder Katherisiervorrichtung nach einem der voranstehenden Ansprüche,
**dadurch gekennzeichnet, daß**
Bedienungselemente zum Ein- und Ausschalten und zur Intensitätssteuerung in der Weise Mehrfachnutzen, daß eine Doppelbetätigung von Bedienungselementen (Ein-/Ausschalter), Intensitätsschalter) dem Ein- und Ausschalten der Durchblutungs-Diagnostiziervorrichtung (9) und der Lautstärke von dessen Schallsignal (22) zugeordnet ist, während eine Einfachbedienung der Bedienungselemente dem Stimualtorstromkreis für Ein- und Ausschalten und Verstärkung und Verringerung der Stimulatorimpulse zugeordnet ist, oder umgekehrt.

7. Punktier- und/oder Katherisiervorrichtung nach einem der vorhergehenden Ansprüche,
**dadurch gekennzeichnet, daß**
sie in ihrem Stimulatorstromkreis (5) in bekannter Weise für Iststrommessung in der Körpergewebestrecke zwischen Hautklebe-Elektrode und Punktierelelektrode ausgelegt und mit Stromkonstanthalter ausgestattet ist.

8. Punktier- und/oder Katherisiervorrichtung nach einem der vorhergehenden Ansprüche,
**dadurch gekennzeichnet, daß**
die Diagnostiziervorrichtung insbesondere Ultraschallsonde, bzw. Doppler-Sonde (29) und Punktiernadelelektrode (31) nach Art eines Zielgerätes (Fig. 3) zusammenfügbar sind.

9. Punktier- und/oder Katheterisiervorrichtung
**gekennzeichnet durch**
eine geteilte Sonde mit mittlerer Aufnahmeöffnung für die Punktiernadelelektrode.
